(19) Europäisches Patentamt
European Patent Office
Office européen des brevets

(11) EP 0 618 904 B1

(12) **EUROPÄISCHE PATENTSCHRIFT**

(45) Veröffentlichungstag und Bekanntmachung des Hinweises auf die Patenterteilung:
**14.04.1999 Patentblatt 1999/15**

(21) Anmeldenummer: **93921881.4**

(22) Anmeldetag: **29.09.1993**

(51) Int Cl.[6]: **C07D 231/38**, A61K 7/13

(86) Internationale Anmeldenummer:
**PCT/EP93/02651**

(87) Internationale Veröffentlichungsnummer:
**WO 94/08971 (28.04.1994 Gazette 1994/10)**

(54) **NEUE N-PHENYLAMINOPYRAZOL-DERIVATE SOWIE MITTEL UND VERFAHREN ZUR FÄRBUNG VON HAAREN**

NOVEL N-PHENYL AMINO PYRAZOLE DERIVATIVES AND MEANS AND PROCESS FOR COLOURING HAIR

NOUVEAUX DERIVES DU N-PHENYLAMINOPYRAZOLE, AINSI QUE COMPOSES ET PROCEDE POUR LA TEINTURE DES CHEVEUX

(84) Benannte Vertragsstaaten:
**DE ES FR GB IT**

(30) Priorität: **16.10.1992 DE 4234886**

(43) Veröffentlichungstag der Anmeldung:
**12.10.1994 Patentblatt 1994/41**

(73) Patentinhaber: **Wella Aktiengesellschaft
64274 Darmstadt (DE)**

(72) Erfinder:
- **NEUNHOEFFER, Hans
D-64367 Mühltal (DE)**
- **GERSTUNG, Stefan
D-64354 Reinheim (DE)**
- **CLAUSEN, Thomas
D-64665 Alsbach (DE)**
- **BALZER, Wolfgang, R.
D-64665 Alsbach (DE)**

(56) Entgegenhaltungen:
**EP-A- 0 375 977** **DE-A- 2 234 476**

- **CHEMICAL ABSTRACTS, vol. 59, no. 2, 1963, Columbus, Ohio, US; abstract no. 1786b, V. VANICEK ET AL 'Oxidative condensation of 1-aryl-3-methyl-4-amino-5-pyrazolones with m-phenylenediamine and its derivatives' & COLLECTION CZECH. CHEM. COMMUNS. Bd. 27 , 1962 Seiten 2796 - 2800**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue substituierte N-Phenylaminopyrazole sowie diese Verbindungen enthaltendene Haarfärbemittel und ein Verfahren zum Färben von Haaren.

In der Regel werden zur Haarfärbung oxidative Haarfärbemittel eingesetzt, bei denen die Farbstoffe durch oxidative Kupplung von geeigneten Entwicklersubstanzen und Kupplersubstanzen im Haarschaft erzeugt werden. So werden beispielsweise in der EP-OS 0 375 977 Oxidationshaarfärbemittel beschrieben, welche durch oxidative Kupplung von bestimmten Diaminopyrazolderivaten (als Entwicklersubstanzen) mit üblichen Kupplersubstanzen erhalten werden.

Es ist bereits bekannt, zum Färben von Haaren Leucoderivate von Indoanilinen zu verwenden. Diese farblosen Verbindungen werden in wäßriger Lösung auf die zu färbende Faser aufgebracht und durch Luft oder ein anderes Oxidationsmittel zu Indoanilinen oxidiert, welche gefärbte Verbindungen darstellen. Die so erhaltenen Färbungen zeigen aufgrund der guten Löslichkeit der Leucoverbindungen Intensitäts- und Haltbarkeitseigenschaften, die denen der Färbungen, die durch direkte Anwendung von Indoanilinen erhalten werden, überlegen sind. So sind zum Beispiel in der DE-OS 22 34 525 und DE-OS 22 34 476 Leucoderivate dieser Verbindungen beschrieben; die hiermit erreichbare Farbintensitäten sind jedoch nicht befriedigend.

Es bestand daher die Aufgabe, neue Leuco-Verbindungen zur Verfügung zu stellen, die durch Oxidation in Farbstoffe mit hohen Intensitäten und guten Haltbarkeitseigenschaften überführt werden können.

Es wurde nun gefunden, daß die N-Phenylaminopyrazolderivate der allgemeinen Formel (I)

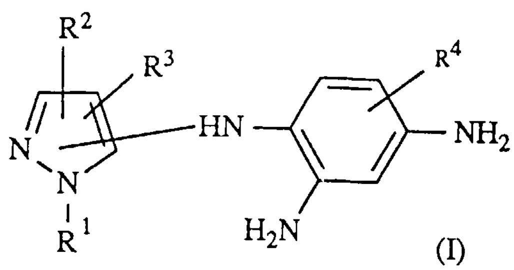 ,

(I)

in der R1 Wasserstoff, $C_1$ bis $C_4$-Alkyl, oder $C_2$ bis $C_4$-Hydroxyalkyl bedeutet,$R^2$ und $R^3$ gleich oder verschieden sein können und Wasserstoff, einen Amino- oder $C_1$ bis $C_4$-Alkyl-Rest darstellen und $R^4$ gleich Wasserstoff,$C_1$ bis $C_4$-Alkyl, Halogen oder $C_1$ bis $C_4$-Alkoxy ist, die gestellte Aufgabe in hervorragender Weise erfüllen. Die Verbindungen der Formel (I) sind daher Gegenstand der Erfindung.

Die Verbindungen der Formel (I) sind auf einfachem Weg über eine Kupplung von Aminopyrazolen der allgemeinen Formel (II), in der $R^1$ die vorstehend angegebene Bedeutung hat und $R^a$ und $R^b$ Wasserstoff, einen $C_1$ bis $C_4$-Alkyl-Rest oder eine Nitrogruppe bedeuten, mit substituierten Dinitrohalogenbenzolen (III) und anschließender Reduktion der erhaltenen (2',4'-Dinitrophenylamino)-pyrazole (IV) herstellbar.

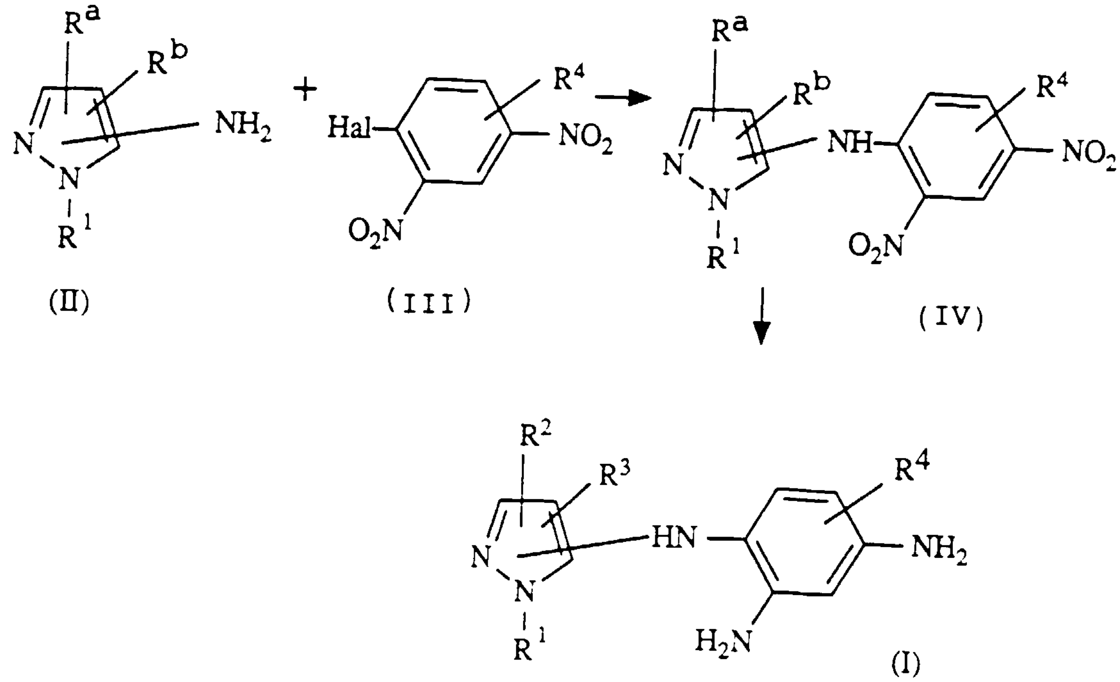

(II) (III) (IV) (I)

Beispiele für geeignete N-Phenylaminopyrazole der Formel (I) sind: 5-Amino-4-(2',4'-diaminophenylamino)-1-methylpyrazol, 4-Amino-5-(2',4'-diaminophenylamino)-1-methylpyrazol, 3-Amino-4-(2',4'-diaminophenylamino)-1-methylpyrazol, 3-(2',4'-Diaminophenylamino)-1-methylpyrazol, 4-(2',4'-Diaminophenylamino)-1-methylpyrazol, 5-(2',4'-Diaminophenylamino)-1-methylpyrazol und 4-(2',4'-Diaminophenylamino)-1,3,5-trimmethylpyrazol.

Die neuen Verbindungen der Formel (I) ergeben nach der Oxidation Farbstoffe mit hoher Farbintensität und guten Haltbarkeitseigenschaften, insbesondere bezüglich Licht-, Reibe- und Waschechtheit.

Ein weiterer Gegenstand der vorliegenden Anmeldung ist daher ein Haarfärbemittel mit einem Gehalt an für Haarfärbemittel üblichen Zusätzen, welches dadurch gekennzeichnet ist, daß es mindestens ein N-Phenylaminopyrazol-Derivat der allgemeinen Formel (I) enthält.

Die Verbindungen der allgemeinen Formel (I) sollen in dem erfindungsgemäßen Mittel in einer Konzentration von 0,001 bis 2 Gewichtsprozent, vorzugsweise in einer Konzentration von 0,01 bis 0,5 Gewichtsprozent enthalten sein, wobei die Verbindungen der allgemeinen Formel (I) auch in Formm ihrer physiologisch verträglichen, wasserlöslichen Salze vorliegen können.

Bei dem erfindungsgemäßen Haarfärbemittel handelt es sich um ein Mittel, das mindestens einen Farbstoff der allgemeinen Formel (I) enthält, oder aber um ein Mittel, welches zusätzlich zu mindestens einem Farbstoff der allgemeinen Formel (I) einen oder mehrere üblicherweise in Haarfärbemitteln verwendete Farbstoffe enthält.

Von diesen Farbstoffen seien beispielsweise die folgenden genannt: Oxidationsfarbstoffe, wie zum Beispiel p- oder m-Phenylendiaminderivate, p- oder m-Aminophenolderivate sowie Resorcin und seine Derivate oder aber direkt auf das Haar aufziehende Farbstoffe, wie zum Beispiel aromatische Nitrofarbstoffe, Triphenylmethanfarbstoffe, Azofarbstoffe und Anthrachinonfarbstoffe.

Das erfindungsgemäße Haarfärbemittel kann in Form einer Lösung, beispielsweise als wäßrige oder wäßrig-alkoholische Lösung, einer Emulsion, einer Creme oder eines Gels vorliegen.

Der pH-Wert dieses Haarfärbemittels liegt im Bereich von 4 bis 11, vorzugsweise im Bereich von 6 bis 9, wobei die Einstellung des gewünschten alkalischen pH-Wertes mit Ammoniak, organischen Aminen, beispielsweise Monoethanolamin, oder Natronlauge erfolgen kann, während zur Einstellung eines sauren pH-Wertes Phosphorsäure oder organische Säuren, wie zum Beispiel Essigsäure, Weinsäure oder Zitronensäure, verwendet werden können.

Selbstverständlich kann das vorstehend beschriebene Haarfärbemittel gegebenenfalls weitere, für Haarfärbemittel übliche Zusätze, wie zum Beispiel Weichmacher, Konservierungsstoffe und Parfümöle, Lösungsmittel wie Wasser, niedere aliphatische Alkohole, beispielsweise Ethanol, Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain, enthalten. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Das Färben der Haare mit dem erfindungsgemmäßen Haarfärbemittel erfolgt üblicherweise durch Aufbringen einer für die Haarfärbung ausreichenden Menge (60 bis 200 g) des Mittels auf die Haare, mit denen es 10 bis 30 Minuten lang im Kontakt bleibt. Anschließend wird das Haar mit Wasser gespült und sodann getrocknet.

Das erfindungsgemäße Haarfärbemittel kann auch vor der Anwendung mit einem Oxidationsmittel, wie zum Beispiel Wasserstoffperoxid oder dessen Additionsverbindungen, wie beispielsweise Harnstoffperoxid oder Carbamidperoxid, vermischt und anschließend in der angegebenen Weise angewendet werden.

Die nachfolgenden Beispiele sollen den Gegenstand der vorliegenden Erfindung näher erläutern, ohne diesen hierauf zu beschränken.

**Herstellungsbeispiele**

**Beispiel 1:**

**Synthese von (2',4'-Dinitrophenylamino)-pyrazolen**

A. Allgemeine Arbeitsvorschrift zur Umsetzung von Aminopyrazolen mit 2,4-Dinitrochlorbenzolen oder 2,4-Dinitroiodbenzolen

Die angegebene Menge des Aminopyrazols wird in 50 ml absolutem Acetonitril oder Ethanol gelöst und mit der angegebenen Menge eines gegebenenfalls substituierten 2,4-Dinitrochlorbenzols oder 2,4-Dinitroiodbenzols versetzt. Man erhitzt 2 Stunden zum Sieden, gibt dann äquimolare Menge Kaliumhydroxidpulver oder Ammoniak hinzu und läßt noch 2 bis 4 Stunden bei Siedetemperatur reagieren. Anschließend dampft man das Lösungsmittel ab, nimmt den Rückstand in 100 ml Wasser auf und extrahiert dreimal mit je 100 ml Essigester. Nach dem Trocknen über Magnesiumsulfat wird eingedampft und an Kieselgel mit Ether chromatographiert.

I. Synthese von 4-(2',4'-Dinitrophenylamino)-1-methyl-5-nitropyrazol

100 mg (0,70 mmol) 4-Amino-1-methyl-5-nitropyrazol werden in Acetonitril analog der allgemeinen Arbeitsvorschrift mit 210 mg (0,70 mmol) 2,4-Dinitroiodbenzol umgesetzt. Nach 2 Stunden werden 40 mg (0,70 mmol) Kaliumhydroxid zugegeben.

Ausbeute: 120 mg (55 Prozent der Theorie) 4-(2'4'-Dinitrophenylammino)-1-methyl-5-nitropyrazol als orange Kristalle mit einem Schmelzpunkt von 156 Grad Celsius (Ether).
60 MHz-$^1$H-NMR ([$D_6$] DMSO):

δ = 10,78 ppm (s, breit, 1H, NH, mit $D_2O$ austauschbar),
8,92 ppm (d, J = 2 Hz, 1H, 3'-H),
8,36 ppm (dd, $J^1$ = 10 Hz, $J^2$ = 2 Hz, 1H, 5'-H),
8,13 ppm (s, 1H, 3-H),
7,65 ppm (d, J = 10 Hz, 1H, 6'-H) und
4,20 ppm (s, 3H, $CH_3$)

MS (70 ev) m/e: 308 [$M^+$].

II. Synthese von 5-(2',4'-Dinitrophenylamino)-1-methyl-4-nitropyrazol

Analog der allgemeinen Arbeitsvorschrift werden 0,55 g (3,87 mmol) 5-Amino-1-methyl-4-nitropyrazol mit 1,14 g (3,87 mmol) 2,4-Dinitroiodbenzol und 210 mg (3,87 mmol) Kaliumhydroxid umgesetzt und aufgearbeitet.

Ausbeute: 0,65 g (55 Prozent der Theorie) 5-(2',4'-Dinitrophenylamino)-1-methyl-4-nitropyrazol als hellgelbe Kristalle mit einem Schmelzpunkt von 182 Grad Celsius (Ether).
60 MHz-$^1$H-NMR ([$D_6$] DMSO):

δ = 10,19 ppm (s, breit, 1H, NH, mit $D_2O$ austauschbar),

8,93 ppm (d, J = 2 Hz, 1H, 3'-H),
8,42 ppm (s, 1H, 3-H),
8,25 ppm (dd, $J^1$ = 10 Hz, $J^2$ = 2 Hz, 1H, 5'H),
6,95 ppm (d, J = 10 Hz, 1H, 6'-H) und
3,80 ppm (s, 3H, $CH_3$)

MS (70 eV) m/e: 308 [$M^+$].

III. Synthese von 4-(2',4'-Dinitrophenylamino)-1-methyl-3-nitropyrazol

Analog der allgemeinen Arbeitsvorschrift werden 100 mg (0,70 mmol)4-(2',4'-Dinitrophenylamino)-1-methyl-3-nitropyrazol mit 210 mg (0,70 mmol) 2,4-Dinitroiodbenzol und 40 mg (0,70 mmol) Kaliumhydroxid umgesetzt und aufgearbeitet.

Ausbeute: 130 mg (60 Prozent der Theorie) 4-(2',4'-Dinitrophenylamino)-1-methyl-3-nitropyrazol als orange Kristalle mit einem Schmelzpunkt von 195 Grad Celsius (Ether).
60 MHz-$^1$H-NMR ([$D_6$]DMSO):

δ = 10,63 ppm (s, breit, 1H, NH, mit $D_2O$ austauschbar),
8,92 ppm (d, J = 2 Hz, 1H, 3'-H),
8,49 ppm (s, 1H, 3-H),
8,30 ppm (dd, $J^1$ = 10 Hz, $J^2$ = 2 Hz, 1H, 5'-H),
7,46 ppm (d, J = 10 Hz, 1H, 6'-H) und
4,03 ppm (s, 3H, $CH_3$)

MS (70 eV) m/e: 308 [$M^+$].

IV. Synthese von 3-(2',4'-Dinitrophenylamino)-1-methyl-pyrazol

Analog der allgemeinen Arbeitsvorschrift werden 2,00 g (20,6 mmol) 3-Amino-1-methylpyrazol mit 4,17 g (20,6 mmol) 2,4-Dinitrochlorbenzol und 1,15 g (20,6 mmol) Kaliumhydroxid umgesetzt und aufgearbeitet.

Ausbeute: 3,20 g (59 Prozent der Theorie) 3-(2',4'-Dinitrophenylamino)-1-methylpyrazol als rote Nadeln mit einem Schmelzpunkt von 150 Grad Celsius (Essigester).
60 MHz-$^1$H-NMR ([$D_6$] DMSO):

δ = 10,15 ppm (s, breit, 1H, NH, mit $D_2O$ austauschbar),
8,84 ppm (d, J = 3 Hz, 1H, 3'-H),
8,33 ppm (dd, $J^1$ = 10 Hz, $J^2$ = 3 Hz, 1H, 5'-H),
8,03 ppm (d, J = 10 Hz, 1H, 6'-H),
7,77 ppm (d, J = 2 Hz, 1H, 5-H),
6,26 ppm (d, J = 2 Hz, 1H, 4-H) und
3,85 ppm (s, 3H, $CH_3$).

MS (70 eV) m/e : 263 [$M^+$]

V. Synthese von 4-(2',4'-Dinitrophenylamino)-1-methylpyrazol

Eine Lösung von 0,50 g (5,15 mmol) 4-Amino-1-methylpyrazol in 50 ml Acetonitril (oder Ethanol) wird mit 2,00 g (9,90 mmol) 2,4-Dinitrochlorbenzol versetzt und 2 Stunden zum Sieden erhitzt. Nach Zugabe von 5 ml konz. Ammoniak wird weitere 2 Stunden unter Rühren zum Sieden erhitzt. Beim Abkühlen fällt das Produkt aus. Es wird abgesaugt, mit Wasser gewaschen und im Vakuum getrocknet.

Ausbeute: 0,64 g (47 Prozent der Theorie) 4-(2',4'-Dinitrophenylamino)-1-methylpyrazol als rote Nadeln mit einem Schmelzpunkt von 149 Grad Celsius (Ethanol).
60 MHz-$^1$H-NMR ([$D_6$] DMSO):

δ = 9,72 ppm (s, breit, 1H, NH, mit $D_2O$ austauschbar),
8,82 ppm (d, J = 3 Hz, 1H, 3'-H),
8,20 ppm (dd, $J^1$ = 10 Hz, $J^2$ = 3 Hz, 1H, 5'-H),
7,93 ppm (s, 1H, 5-H),

7,53 ppm (s, 1H, 3-H),
7,15 ppm (d, J = 10 Hz, 1H, 6'-H) und
3,90 ppm (s, 3H, $CH_3$)

MS (70 eV) m/e: 263 [$M^+$].

VI. Synthese von 5-(2',4'-Dinitrophenylamino)-1-methylpyrazol

Analog der allgemeinen Arbeitsvorschrift werden 1,60 g (16,5 mmol) 5-Amino-1-methylpyrazol mit 3,34 g (16,5 mmol) 2,4-Dinitrochlorbenzol und 0,90 g (16,5 mmol) Kaliumhydroxid umgesetzt und aufgearbeitet.
Ausbeute: 1,52 g (35 Prozent der Theorie) 5-(2',4'-Dinitrophenylamino)-1-methylpyrazol als gelbe Kristalle mit einem Sublimationspunkt von 175 Grad Celsius (Ether).
60 MHz-$^1$H-NMR ([$D_6$] DMSO):

δ = 9,95 ppm (s, breit, 1H, NH, mit $D_2O$ austauschbar),
8,90 ppm (d, J = 3 Hz, 1H, 3'-H),
8,30 ppm (dd, $J^1$ = 10 Hz, $J^2$ = 3 Hz, 1H, 5'-H),
7,57 ppm (d, J = 2 Hz, 1H, 1H, 3-H),
6,83 ppm (d, J = 10 Hz, 1H, 6'-H),
6,31 ppm (d, J = 2 Hz, 1H, 4-H) und
3,69 ppm (s, 3H, $CH_3$)

MS (70 eV) m/e: 263 [$M^+$].

VII. Synthese von 4-(2',4'-Dinitrophenylamino)-1,3,5-trimethyl-pyrazol

1,00 g (4,15 mmol) 4-Amino-1,3,5-trimethylpyrazolhydrosulfat-hydrat werden in 50 ml Ethanol mit 10 ml konzentriertem Ammoniak und 1,26 g (6,22 mmol) 2,4-Dinitrochlorbenzol versetzt und 2 Stunden zum Sieden erhitzt. Die Aufarbeitung erfolgt analog der allgemeinen Arbeitsvorschrift.
Ausbeute: 0,87 g (72 Prozent der Theorie) 4-(2',4'-Dinitrophenylamino)-1,3,5-trimethylpyrazol als orange Kristalle mit einem Schmelzpunkt von 153 Grad Celsius (Ether).
60 MHz-$^1$H-NMR ([$D_6$] DMSO):

δ = 9,60 ppm (s, breit, 1H, NH, mit $D_2O$ austauschbar),
8,89 ppm (d, J = 3 Hz, 1H, 3'-H),
8,81 ppm (dd, $J^1$ = 10 Hz, $J^2$ = 3 Hz, 1H, 5'-H),
6,77 ppm (d, J = 10 Hz, 1H, 6'-H),
3,72 ppm (s, 3H, 1-$NCH_3$),
2,10 ppm (s, 3H, 3(5)-$CH_3$) und
1,97 ppm (s, 3H, 5(3)-$CH_3$)

MS (70 eV) m/e: 291[$M^+$].

**Beispiel 2:**

**Synthese von (2',4'-Diaminophenylamino)-pyrazolen**

A. Allgemeine Arbeitsvorschrift zur Hydrierung in Schwefelsäure

Die angegebene Menge des Nitropyrazols wird in einer Lösung der äquimolaren Menge konzentrierter Schwefelsäure in 25 ml Wasser mit 100 mg Palladium/ Aktivkohle-Katalysator versetzt und bei Normaldruck und Raumtemperatur unter kräftigem Schütteln in einem Hydrierkolben 2 bis 6 Stunden hydriert. Zur Beschleunigung der Reaktion von sehr schlecht löslichen Substanzen gibt man 5 ml Ethanol zur Reaktionsmischung. Nach Beendigung der Reaktion wird unter Stickstoffatmosphäre der Katalysator abgesaugt und die Mutterlauge bis zur Trockene eingedampft. Der Rückstand wird mit 5 ml absolutem Ethanol versetzt, wobei Kristallisation eintritt. Man saugt das Produkt ab, wäscht mit Ethanol und trocknet im Vakuum.

I. Synthese von 5-Amino-4-(2',4'-diaminophenylamino)-1-methylpyrazol

100 mg (0,33 mmol) 4-(2',4'-Dinitrophenylamio)-1-methyl-5-nitro-pyrazol werden in einer Lösung von 35 mg (0,33 mmol) konzentrierter Schwefelsäure in 25 ml Wasser 4 Stunden lang hydriert.
Ausbeute: 90 mg (87 Prozent der Theorie) 5-Amino-4-(2',4'-diaminophenylamino)-1-methylpyrazol hydrosulfat als farblose Kristalle mit einem Zersetzungspunkt von 165 Grad Celsius (Ethanol).

II. Synthese von 4-Amino-5-(2',4'-diaminophenylamino)-1-methyl-pyrazol

0,50 g (1,62 mmol) 5-(2',4'-dinitrophenylamino)-1-methyl-4-nitropyrazol werden in einer Lösung von 165 mg (1,62 mmol) konzentrierter Schwefelsäure in 50 ml Wasser hydriert.
Ausbeute: 340 mg (45 Prozent der Theorie) 4-Amino-5-(2',4'-diaminophenylamino)-1-methyl-pyrazol-dihydrosulfat-trihydrat als schwarze Kristalle mit einem Zersetzungspunkt von 170 Grad Celsius (Ethanol).
300 MHz-$^1$H-NMR ([$D_6$] DMSO/$D_2O$):

$\delta$ = 7,39 ppm (s, 1H, 3-H),
6,55 ppm (s, 1H, 3'-H),
6,32 ppm (d, J = 8,0 Hz, 1H, 5'-H),
6,07 ppm (d, J = 8,0 Hz, 1H, 6'-H),
4,92 ppm (s, breit, 17H, NH, $NH_2$, $H_2SO_4$, $H_2O$) und
3,44 ppm (s, 3 H, $CH_3$)

MS (70 eV) m/e: 218 [$M^+$].

III. Synthese von 3-Amino-4-(2',4'-diaminophenylamind)-1-methylpyrazol

70 mg (0,23 mmol) 4-(2',4'-Dinitrophenylamino)-1-methyl-3-nitro-pyrazol werden in einer Lösung von 25 mg (0,23 mmol) konzentrierter Schwefelsäure in 20 ml Wasser hydriert.
Ausbeute: 55 mg (97 Prozent der Theorie) 3-Amino-4-(2',4'-diaminophenylamino)-1-methylpyrazol-hydrosulfat als farblose, hygroskopische Kristalle mit einem Zersetzungspunkt von 168 Grad Celsius (Ethanol).

IV. Synthese von 3-(2',4'-Diaminophenylamino)-1-methylpyrazol

1,00 g (3,80 mmol) 3-(2',4'-Dinitrophenylamino)-1-methylpyrazol werden in einer Lösung von 380 mg (3,80 mmol) konzentrierter Schwefelsäure in 50 ml Wasser hydriert.
Ausbeute: 0,57g(50 Prozent der Theorie) 3-(2',4'-Diaminophenylamino)-1-methylpyrazol-hydrosulfat als farblose, hygroskopische Kristalle mit einem Zersetzungspunkt von 152 Grad Celsius (Ethanol).
MS (70 eV) m/e: 203 [$M^+$].

V. Synthese von 4-(2',4'-Diaminophenylamino)-1-methylpyrazol

0,50 g (1,90 mmol) 4-(2',4'-Dinitrophenylamino)-1-methylpyrazol werden in einer Lösung von 190 mg (1,90 mmol) konzentrierter Schwefelsäure in 30 ml Wasser hydriert.
Ausbeute: 0,39 g (69 Prozent der Theorie) 4-(2',4'-Diaminophenylamino)-1-methylpyrazol-hydrosulfat als beige, hygroskopische Kristalle mit einem Zersetzungspunkt von 162 bis 164 Grad Celsius (Ethanol).
MS (70 eV)m/e: 203 [$M^+$],

VI. Synthese von 5-(2',4'-Diaminophenylamino)-1-methylpyrazol

140 mg (0,53 mmol) 5-(2',4'-Dinitrophenylamino)-1-methylpyrazol werden in einer Lösung von 55 mg (0,53 mmol) konzentrierter Schwefelsäure in 20 ml Wasser hydriert.
Ausbeute: 100 mg (63 Prozent der Theorie) 5-(2',4'-Diaminophenylamino)-1-methylpyrazolhydrosulfat als farblose, hygroskopische Kristalle mit einem Zersetzungspunkt von 183 Grad Celsius (Ethanol).
MS (70 eV)m/e: 203 [$M^+$].

VII. Synthese von 4-(2',4'-Diaminophenylamino)-1,3,5-trimethylpyrazol

400 mg (1,37 mmol) 4-(2',4'-Dinitrophenylamino)-1,3,5-trimethylpyrazol werden in einer Lösung von 140 mg

(1,37 mmol) konzentrierter Schwefelsäure in 20 ml Wasser hydriert.

Ausbeute: 380 mg (84 Prozent der Theorie) 4-(2',4'-Diaminophenylamino)-1,3,5-trimethylpyrazol-hydrosulfat als farblose, hygroskopische Kristalle mit einem Zersetzungspunkt von 165 Grad Celsius (Ethanol).

MS (70 eV)m/e: 231 [$M^+$].

Für sämtliche NMR-Spektren gilt:
s = Singulett, d = Dublett

**Haarfärbebeispiele**

**Beispiel 3 bis 9:**

Es wurden Haarfärbelösungen folgender Zusammensetzung hergestellt:

| | |
|---|---|
| 2,50 g | Farbstoff nach Beispiel 2/I bis VII |
| 10,00 g | Laurylalkoholdiglycolethersulfat-Natriumsalz (28-prozentige wäßrige Lösung) |
| 87,50 g | Wasser |
| 100,00 g | |

Die Haarfärbelösungen wurden auf zu 90 Prozent ergraute, menschliche Haare aufgetragen und 30 Minuten bei 40 Grad Celsius einwirken gelassen. Das Haar wurde mit Wasser gespült und getrocknet. Die resultierenden Färbungen sind in Tabelle 1 dargestellt.

Tabelle 1

| Beispiel | Farbstoff aus Beispiel | Farbe |
|---|---|---|
| 3 | 2/I | leuchtend violett |
| 4 | 2/II | braun |
| 5 | 2/III | violett |
| 6 | 2/IV | rot-braun |
| 7 | 2/V | rot-braun |
| 8 | 2/VI | rot-braun |
| 9 | 2/VII | braun |

Alle in der vorliegenden Patentanmeldung angegebenen Prozentzahlen stellen, sofern nicht anders angegeben, Gewichtsprozente dar.

**Patentansprüche**

1. N-Phenylaminopyrazol-Derivat der allgemeinen Formel (I)

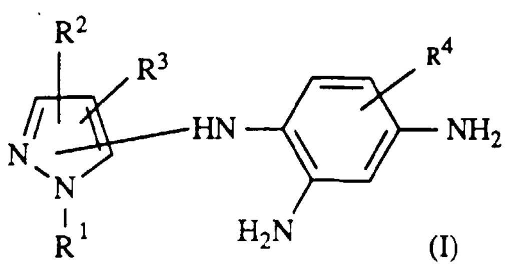

,

(I)

in der $R^1$ Wasserstoff, $C_1$- bis $C_4$-Alkyl oder $C_2$- bis $C_4$-Hydroxyalkyl bedeutet, $R^2$ und $R^3$ gleich oder verschieden sein können und Wasserstoff, einen Amino- oder $C_1$- bis $C_4$-Alkylrest darstellen und $R^4$ gleich Wasserstoff, $C_1$- bis $C_4$-Alkyl, Halogen oder $C_1$- bis $C_4$-Alkoxy ist.

**2.** 5-Amino-4-(2',4'-diaminophenylamino)-1-methylpyrazol.

**3.** 4-Amino-5-(2',4'-diaminophenylamino)-1-methylpyrazol.

**4.** 3-Amino-4-(2',4'-diaminophenylamino)-1-methylpyrazol.

**5.** 3-(2',4'-Diaminophenylamino)-1-methylpyrazol.

**6.** 4-(2',4'-Diaminophenylamino)-1-methylpyrazol.

**7.** 5-(2',4'-Diaminophenylamino)-1-methylpyrazol.

**8.** 4-(2',4'-Diaminophenylamino)-1,3,5-trimethylpyrazol.

**9.** Haarfärbemittel mit einem Gehalt an für Haarfärbemittel üblichen Zusätzen, dadurch gekennzeichnet, daß es mindestens ein N-Phenylaminopyrazol-Derivat der allgemeinen Formel (I) enthält.

**10.** Mittel nach Anspruch 9, dadurch gekennzeichnet, daß die N-Phenylaminopyrazol-Derivate der allgemeinen Formel (I) in einer Konzentration von 0,001 bis 2,0 Gewichtsprozent enthalten sind.

**11.** Mittel nach Anspruch 9 oder 10, dadurch gekennzeichnet, daß der pH im Bereich von 4 bis 11 liegt.

**12.** Mittel nach einem der Ansprüche 9 bis 11, dadurch gekennzeichnet, daß es in Form einer wäßrigen oder wäßrig-alkoholischen Lösung, einer Emulsion, einer Creme oder eines Gels vorliegt.

**13.** Mittel nach einem der Ansprüche 9 bis 12, dadurch gekennzeichnet, daß es zusätzlich einen oder mehrere Oxidationsfarbstoffe oder direkt auf das Haar aufziehende Farbstoffe enthält.

**14.** Verfahren zum Färben von Haaren, dadurch gekennzeichnet, daß eine für die Haarfärbung ausreichende Menge eines Mittels nach einem der Ansprüche 9 bis 13 auf das Haar aufgebracht wird und nach einer Einwirkungszeit von 10 bis 30 Minuten das Haar mit Wasser gespült und getrocknet wird.

**15.** Verfahren nach Anspruch 14, dadurch gekennzeichnet, daß das Mittel nach einem der Ansprüche 9 bis 13 vor der Anwendung mit einem Oxidationsmittel vermischt wird.

**Claims**

**1.** N-phenylaminopyrazole derivative of the general formula (I)

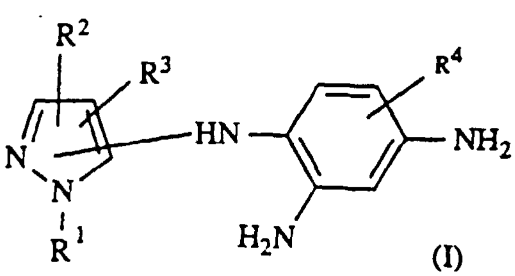

,

(I)

in which $R^1$ signifies hydrogen, $C_1$ to $C_4$ alkyl or $C_2$ to $C_4$ hydroxyalkyl, $R^2$ and $R^3$ can be identical or different and represent hydrogen, an amino or $C_1$ to $C_4$ alkyl residue, and $R^4$ is hydrogen, $C_1$ to $C_4$ alkyl, halogen or $C_1$ to $C_4$ alkoxy.

**2.** 5-amino-4-(2',4'-diaminophenylamino)-1-methylpyrazole.

**3.** 4-amino-5-(2',4'-diaminophenylamino)-1-methylpyrazole.

**4.** 3-amino-4-(2',4'-diaminophenylamino)-1-methylpyrazole.

**5.** 3-(2',4'-diaminophenylamino)-1-methylpyrazole.

**6.** 4-(2',4'-diaminophenylamino)-1-methylpyrazole.

**7.** 5-(2',4'-diaminophenylamino)-1-methylpyrazole.

**8.** 4-(2',4'-diaminophenylamino)-1,3,5-trimethylpyrazole.

**9.** Hair colouring agent containing additives conventional for hair colouring agents, characterised in that it contains at least one N-phenylaminopyrazole derivative of the general formula (I).

**10.** Agent according to Claim 9, characterised in that the N-phenylaminopyrazole derivatives of the general formula (I) are present in a concentration of between 0.001 and 2.0 weight percent.

**11.** Agent according to Claim 9 or 10, characterised in that the pH is in the range of from 4 to 11.

**12.** Agent according to any one of Claims 9 to 11, characterised in that it is in the form of an aqueous or aqueous-alcoholic solution, an emulsion, a cream or a gel.

**13.** Agent according to any one of Claims 9 to 12, characterised in that it additionally contains one or more oxidative dyes or direct-acting dyes.

**14.** Method of colouring hair, characterised in that an amount of an agent according to any one of Claims 9 to 13 which is sufficient for hair colouring is applied to the hair and, after a reaction time of 10 to 30 minutes, the hair is rinsed with water and dried.

**15.** Method according to Claim 14, characterised in that the agent according to any one of Claims 9 to 13 is mixed before use with an oxidising agent.

**Revendications**

**1.** Dérivé de N-phénylaminopyrazol de formule générale (I)

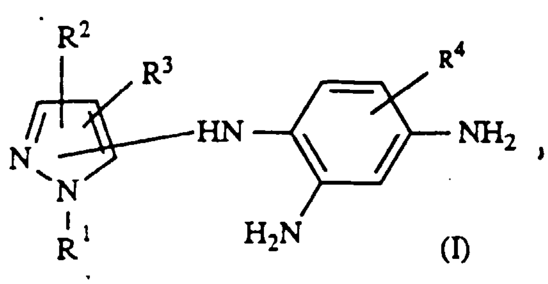

(I)

dans laquelle $R^1$ représente l'hydrogène, un groupe alkyle en $C_1$-$C_4$, ou un groupe hydroxyalkyle en $C_2$-$C_4$, $R^2$ et $R^3$, peuvent être identiques ou différents et représentent l'hydrogène, un reste amino ou un reste alkyle en $C_1$-$C_4$, et $R^4$ est l'hydrogène, un groupe alkyle en $C_1$-$C_4$, un halogène ou un groupe alcoxy en $C_1$-$C_4$.

**2.** 5-amino-4-(2',4'-diaminophénylamino)-1-méthylpyrazol.

**3.** 4-amino-5-(2',4'-diaminophénylamino)-1-méthylpyrazol.

**4.** 3-amino-4-(2',4'-diaminophénylamino)-1-méthylpyrazol.

5. 3-(2',4'-diaminophénylamino)-1-méthylpyrazol.

6. 4-(2',4'-diaminophénylamino)-1-méthylpyrazol.

7. 5-(2',4'-diaminophénylamino)-1-méthylpyrazol.

8. 4-(2',4'-diaminophénylamino)-1,3,5-triméthylpyrazol.

9. Agent de teinture capillaire comprenant une teneur en additifs usuels pour ces agents, caractérisé en ce qu'il contient au moins un dérivé de N-phénylaminopyrazol de formule générale (I).

10. Agent selon la revendication 9, caractérisé en ce que les dérivés de N-phénylaminopyrazol de formule générale (I), sont présents à raison de 0,001 à 2,0% en poids.

11. Agent selon la revendication 9 ou 10, caractérisé en ce que le pH se situe dans la zone de 4 à 11.

12. Agent selon l'une des revendications 9 à 11, caractérisé en ce qu'il se présente sous la forme d'une solution aqueuse ou hydro-alcoolique, d'une émulsion, d'une crème ou d'un gel.

13. Agent selon l'une des revendications 9 à 12, caractérisé en ce qu'il contient en plus un ou plusieurs colorant par oxydation ou des colorants se fixant directement sur les cheveux.

14. Procédé pour colorer les cheveux, caractérisé en ce qu'on apporte sur les cheveux pour la teinture de ceux-ci, des quantités suffisantes d'un agent selon une des revendications 9 à 13, et qu'après une durée d'action de 10 à 30 minutes, on rince les cheveux à l'eau et on les sèche.

15. Procédé selon la revendication 14, caractérisé en ce que l'agent selon l'une des revendications 9 à 13, est mélangé avant utilisation avec un agent d'oxydation.